# EUROPÄISCHE PATENTSCHRIFT

(11) **EP 3 416 601 B1**
(45) Veröffentlichungstag und Bekanntmachung des Hinweises auf die Patenterteilung: **11.08.2021**
(21) Anmeldenummer: 17704473.2
(22) Anmeldetag: 13.02.2017
(51) Int. Cl.: A61F 13/49, A61F 13/56, A61F 13/64

(54) **INKONTINENZWEGWERFWINDEL**
DISPOSABLE INCONTINENCE DIAPER
COUCHE JETABLE POUR INCONTINENCE

(30) Priorität: 16.02.2016 DE 102016102684
(43) Veröffentlichungstag der Anmeldung: 26.12.2018
(73) Patentinhaber: Paul Hartmann AG, 89522 Heidenheim (DE)
(72) Erfinder: OSTERTAG, Wolfgang, 89547 Gerstetten (DE); EBERT, Anselm, 97204 Höchberg (DE); KESSELMEIER, Rüdiger, 89233 Burlafingen (DE); DRUMEVA-EBERIUS, Albena, 89129 Langenau (DE)
(86) Internationale Anmeldenummer: PCT/EP2017/053106
(87) Internationale Veröffentlichungsnummer: WO 2017/140604

(56) Entgegenhaltungen:
- WO-A1-2004/021949
- WO-A1-2007/035903
- WO-A1-2007/149017
- WO-A1-2009/082290
- WO-A1-2012/069166
- WO-A1-2012/069166
- WO-A1-2017/114695
- DE-A1-102005 048 868

## Beschreibung

Die Erfindung betrifft eine Inkontinenzwegwerfwindel, mit einem einen Saugkörper aufweisenden Hauptteil mit einer Längsrichtung und einer Querrichtung, umfassend einen Vorderbereich mit vorderen seitlichen Längsrändern, einen Rückenbereich mit hinteren seitlichen Längsrändern und einen dazwischen angeordneten, zwischen den Beinen eines Benutzers zu liegen kommenden Schrittbereich, und mit beidseits an den Rückenbereich angefügten hinteren Seitenabschnitten, welche sich in der Querrichtung der Inkontinenzwegwerfwindel über die hinteren seitlichen Längsränder des Hauptteils hinaus erstrecken und im Bereich ihres in Querrichtung freien Endes jeweils wenigstens ein Verschlussmittel tragen, wohingegen an den Vorderbereich keine Seitenabschnitte angefügt sind, sondern die vorderen seitlichen Längsränder des Hauptteils einen frei endenden Längsrand der Windel bilden, wobei die hinteren Seitenabschnitte zum Anlegen und Schließen der Inkontinenzwegwerfwindel an einen Benutzer jeweils entlang einer Umfangsrichtung um den Körper des Benutzers herumlegbar und in überlappende Anordnung mit einer Außenseite des Vorderbereichs bringbar sind, an der sie dann über das jeweilige Verschlussmittel jeweils lösbar anhaftbar sind, wobei die hinteren Seitenabschnitte in eben ausgebreitetem jedoch nicht gedehnten Zustand eine Erstreckung Q in der Querrichtung über den jeweiligen hinteren seitlichen Längsrand hinaus aufweisen, wobei eine Längsmittelachse LM der hinteren Seitenabschnitte die Erstreckung Q halbiert, und wobei die hinteren Seitenabschnitte innerhalb dieser Erstreckung Q in der Querrichtung elastisch dehnbar sind und hierfür einen in der Querrichtung und in der Längsrichtung erstreckten elastischen oder elastifizierten Bereich aufweisen, wobei diese Erstreckung der hinteren Seitenabschnitte in der Querrichtung einen an den hinteren seitlichen Längsrand anschließenden proximalen Abschnitt und einen an den proximalen Abschnitt anschließenden, frei endenden distalen Abschnitt umfasst.

Bei einer derartigen Inkontinenzwegwerfwindel handelt es sich um eine so genannte T-Form-Windel, die sich von anderen Windelkonzepten grundlegend unterscheidet. Bei derartigen T-Form-Windeln, wie sie beispielsweise in WO 2007/035903 A1 beschrieben sind, sind nur im Rückenbereich Seitenabschnitte an den Hauptteil angefügt, während die Windel im Vorderbereich keine zusätzlichen angefügten Seitenabschnitte aufweist sondern durch jeweilige seitliche Längsränder des Hauptteils begrenzt ist. Die hinteren Seitenabschnitte sind in der Längsrichtung der Windel zumeist ausladend, jedoch in der Regel kürzer als die Erstreckung der hinteren seitlichen Längsränder des Hauptteils im Rückenbereich. In der Querrichtung sind die hinteren Seitenabschnitte von T-Form-Windeln derart erstreckt, dass sie in Überlappung mit der Außenseite des Vorderbereichs des Hauptteils gebracht werden können, damit die im Bereich der jeweiligen freien Enden der hinteren Seitenabschnitte vorgesehenen Verschlussmittel auf die Außenseite des Hauptteils der Windel geschlossen werden können. Eine derartige T-Form Windel zeigt auch WO2009082290A1. Die Seitenabschnitte sind im Wesentlichen über ihre gesamte Quererstreckung elastifiziert. Hingegen erstrecken sich die elastischen Zonen jeweils nur über einen Teil der Längserstreckung der Seitenabschnitte. Die WO2004021949 offenbart ein Ohrenelement zur Anfügung an einen absorbierenden Hygieneartikel. Das Ohrenelement weist einen ersten, äußeren rechteckigen Bereich und einen zweiten, inneren konturierten Bereich auf. Die Elastifizierung ist vorzugsweise in dem äußeren rechteckigen Bereich angeordnet, kann sich aber auch in den konturierten Bereich hineinerstrecken.

Mit WO2012069166A1 sind darüber hinaus Windeln mit im Rückenbereich und im Vorderbereich, jeweils beidseitig, angeordneten Seitenabschnitten bekannt geworden, bei denen die hinteren Seitenabschnitte eine höhere Dehnbarkeit aufweisen als die vorderen Seitenabschnitte. Eine T-Form Windel zeigt auch WO 2017/114695 A1 (Art. 54(3) EPÜ).

Im Unterschied hierzu sind sogenannte Gürtelwindeln in großem Umfang bekannt geworden, bei denen sich beidseits vom Rückenbereich des Windelhauptteils in Querrichtung sehr lange Gürtelabschnitte weg erstrecken, die derart bemessen sind, dass sie um den gesamten Bauchumfang des Benutzers herum auf sich selbst geschlossen werden können. Bei Anlegen einer Gürtelwindel wird das Produkt von hinten gegen den Hüft- oder Rückenbereich des Benutzers gelegt, und es werden sodann die beiden Gürtelabschnitte auf der Bauchseite des Benutzers direkt aufeinander geschlossen. Hierbei werden keinerlei Schrägzugkräfte auf den Gürtel oder den Hauptteil eingeleitet, sondern es wird eine lediglich in Hüftumfangsrichtung wirkende Kraft auf die beiden Gürtelabschnitte ausgeübt. Danach wird der Windelhauptteil zwischen den Beinen des Benutzers hervorgeholt und mittels weiterer Verschlusselemente zumeist gegen die Außenseite des zuvor geschlossenen Gürtels positioniert und fixiert. Es wurden auch bereits Bereiche der Gürtelabschnitte elastisch ausgebildet, z.B. EP 2 029 079 B1.

Bei hier in Rede stehenden T-Form-Windeln, bei denen die hinteren Seitenabschnitte auf die Außenseite des Vorderbereichs des Windelhauptteils geschlossen werden, werden beim Schließen häufig starke Zugkräfte in die Seitenabschnitte und in den Windelhauptteil eingeleitet, weil der Benutzer oder eine Pflegeperson beim Anlegen der Windel bestrebt ist, eine deutliche Überlappungssituation zwischen den hinteren Seitenabschnitten und dem Vorderbereich des Hauptteils herzustellen und hierbei gleichzeitig die für einen Festsitz der Windel erforderliche Zugkraft in das Gesamtsystem einzubringen, so dass die Windel auch dauerhaft am Benutzer gehalten wird. Hierbei kommt es in der Pflegesituation auch zur Ausübung von Schrägzugkräften, also von Zugkräften, die neben einer Komponente in Querrichtung eine Komponente in Längsrichtung aufweisen und im Fügebereich der hinteren Seitenabschnitte an den Hauptteil zu kritischen Zuständen führen. Es kommt dort häufig zum Einreißen der Seitenabschnitte oder zum Auftrennen der Fügeverbindung. Dieses Problem stellt sich bei Gürtelwindeln und auch bei Windeln mit hinten und vorn angefügten Seitenabschnitten in bedeutend geringerem Umfang und Ausmaß. Bei Gürtelwindeln ist die Kinematik des Anlegens eine völlig andere, und bei Windeln mit hinteren und vorderen Seitenabschnitten wird eine typische Anlegesituation bereits durch das Überlappen der jeweiligen hinteren und vorderen Seitenabschnitte hergestellt.

Der vorliegenden Erfindung liegt die Aufgabe zu Grunde, eine T-förmige Inkontinenzwegwerfwindel der eingangs genannten Art dahingehend zu verbessern, dass beim Anlegen der Windel an den Körper des Benutzers auftretende Zugkräfte, insbesondere Schrägzugkräfte in geringerem Maße zu Beschädigungen der Windelmaterialien führen, wobei gleichwohl eine kostengünstige Herstellbarkeit des Artikels realisiert werden soll.

Diese Aufgabe wird bei einer Inkontinenzwegwerfwindel der genannten Art erfindungsgemäß dadurch gelöst, dass der jeweilige elastische oder elastifizierte Bereich der hinteren Seitenabschnitte vollständig innerhalb des proximalen Abschnitts angeordnet ist und an den zugeordneten hinteren seitlichen Längsrand heranreicht oder einen Abstand von dem hinteren seitlichen Längsrand in der Querrichtung von höchstens 30 mm aufweist, und dass die jeweiligen hinteren Seitenabschnitte in dem gesamten distalen Abschnitt im wesentlichen undehnbar ausgebildet sind. Hierbei gilt im Rahmen der vorliegenden Erfindung definitionsgemäß, dass der proximale Abschnitt sich in Querrichtung ausgehend von dem hinteren seitlichen Längsrand über eine Länge erstreckt, die 65% der Erstreckung Q eines hinteren Seitenabschnitts beträgt. Das bedeutet, dass der distale Abschnitt sich über eine Länge erstreckt, welche 35 % der Erstreckung Q beträgt.

Mit der vorliegenden Erfindung wurde erkannt, dass elastisch dehnbare Bereiche bei den hinteren Seitenabschnitten zu keiner Verbesserung beim Anlegen der Windel beitragen, wenn sie in der Querrichtung allzu weit entfernt von dem Rückenbereich des Windelhauptteils angeordnet sind. Sie führen im Gegenteil dazu, dass der Benutzer oder eine Pflegeperson sich genötigt fühlt, eine noch größere Zugkraft und Dehnung aufzuwenden. Es wurde festgestellt, dass auf dehnbare, insbesondere elastisch dehnbare Bereiche in dem jeweiligen distalen Abschnitt der hinteren Seitenabschnitte ersatzlos verzichtet werden kann, was die Kosten der Herstellung infolge der Reduzierung der zumeist teuren elastischen oder elastifizierten Materialien reduziert.

Wenn in der vorliegenden Anmeldung im Zusammenhang mit den hinteren Seitenabschnitten von den Eigenschaften "elastisch dehnbar", "dehnbar" oder "undehnbar" die Rede ist, so ist die jeweilige Eigenschaft stets in der Querrichtung der Inkontinenzwegwerfwindel gemeint. Im Hinblick auf eine quantifizierbare Abgrenzung elastisch dehnbarer Bereiche von undehnbaren oder nicht elastisch dehnbaren Bereichen wird auf die weiter unten beschriebenen Mess- oder Testmethoden verwiesen.

Es erweist sich weiter als vorteilhaft, wenn der jeweilige elastische oder elastifizierte Bereich der hinteren Seitenabschnitte an den zugeordneten hinteren seitlichen Längsrand heranreicht, jedoch ohne den hinteren seitlichen Längsrand zu überlappen. In Weiterbildung dieses Erfindungsgedankens beträgt der Abstand eines jeweiligen elastischen oder elastifizierten Bereichs der hinteren Seitenabschnitte von dem hinteren seitlichen Längsrand in der Querrichtung höchstens 20 mm, insbesondere höchstens 10 mm. Dies eröffnet die Möglichkeit, dass im unmittelbaren Übergang des außerhalb des Hauptteils liegenden Bereichs des Seitenabschnitts zu einem mit dem Hauptteil überlappenden Bereich des Seitenabschnitts keine dehnbaren Seitenabschnittmaterialien vorgesehen sind, was die Stabilität der Fügeverbindung erhöht.

Daher erweist es sich auch als vorteilhaft, wenn die beiden hinteren Seitenabschnitte mit einem undehnbaren den Hauptteil überlappenden Bereich an den Hauptteil unlösbar angefügt sind.

Es wird weiter vorgeschlagen, die Seitenabschnitte so auszubilden, dass bei Einleitung üblicher die Gebrauchssituation simulierender Kräfte in der Querrichtung der jeweilige elastische oder elastifizierte Bereich in der Querrichtung um wenigstens 70 %, insbesondere um wenigstens 80 %, weiter insbesondere um wenigstens 90 % dehnbar ist. Hierbei ist eine Dehnbarkeit gemeint, die nicht einhergeht mit einer dauerhaften signifikanten plastischen Verformung oder gar Materialschädigung sondern eine elastische Zurückstellung der gedehnten Seitenabschnitte zulässt und bewirkt. Die Werte werden durch entsprechende Anwendung der weiter unten beschriebenen Mess- oder Testmethoden bestimmt.

Nach einer bevorzugten Ausführungsform ist vorgesehen, dass der elastische oder elastifizierte Bereich der hinteren Seitenabschnitte die Längsmittelachse LM eines jeweiligen hinteren Seitenabschnittes überfängt, sich also in der Querrichtung über die Längsmittelachse LM hinaus in Richtung auf den freien Längsrand der hinteren Seitenabschnitte erstreckt. Vorzugsweise beträgt das Maß der Erstreckung über die Längsmittelachse LM hinaus höchsten 30 mm, insbesondere höchstens 20 mm, weiter insbesondere mindestens 5 mm.

Der Hauptteil umfasst vorzugsweise ein zumindest bereichsweise flüssigkeitsdurchlässiges Topsheet und ein zumindest bereichsweise flüssigkeitsundurchlässiges Backsheet, welche den Saugkörper sandwichartig umgeben. Hierzu erstrecken sich Topsheet und Backsheet zumindest in Querrichtung, vorzugsweise auch in Längsrichtung über die Konturränder des Saugkörpers hinaus, um einen entsprechenden Überhang zu bilden. In dem Überhang sind Topsheet und Backsheet vorzugsweise zumindest bereichsweise miteinander verbunden, insbesondere durch an sich bekannte Fügeverfahren wie Schweißen, Siegeln oder Kleben.

Der Hauptteil weist in dem Schrittbereich beidseitig an einen jeweiligen Längsrand des Schrittbereichs angrenzend je einen in Längsrichtung der Inkontinenzwegwerfwindel erstreckten elastischen oder elastifizierten Abschnitt, mithin einen elastischen oder elastifizierten Beinöffnungsabschnitt auf. "In Längsrichtung" bedeutet hierbei, dass der elastische oder elastifizierte Beinöffnungsabschnitt zumindest eine Komponente in Längsrichtung aufweist, mithin auch schräg oder gekrümmt zur Längsrichtung verlaufen kann.

Vorzugsweise sind hierzu dem Fachmann an sich bekannte elastische Fäden (Lycra® o.Ä.) in vorgespanntem Zustand an den Hauptteil bildenden Materialien fixiert, vorzugsweise an Top- und/oder Backsheet des Hauptteils, insbesondere in einem Bereich, in dem Top- und/oder Backsheet einen Überhang außerhalb der Konturränder des Saugkörpers bilden. Ein elastischer oder elastifizierter Beinöffnungsabschnitt kann nach einer Variante auch durch flachmaterial- oder bandförmige Materialien wie elastische Bänder, Folien, Vliesstoffe oder Schaumstoffe gebildet sein.

Der Saugkörper ist geeignet und dazu bestimmt Körperflüssigkeiten, insbesondere Urin aufzunehmen und dauerhaft zu speichern. Der Saugkörper kann hierzu vorteilhaft superabsorbierendes Polymermaterial (SAP) enthalten, insbesondere zu 5-100 Gewichtsprozent, vorzugsweise zu 10-95 Gewichtsprozent, weiter vorzugsweise zu 15-90 Gewichtsprozent ganz besonders bevorzugt zu 20-80 Gewichtsprozent. Typischerweise kann das SAP-Material zumindest das 15-fache seines Gewichts an 0,9 gewichtsprozentiger Kochsalzlösung absorbieren (gemessen nach EDANA Testmethode ERT 441.2-02).

Das SAP-Material kann beispielsweise partikelförmig oder faserförmig oder blatt- oder schaumförmig ausgebildet sein.

Der Saugkörper kann weitere Materialien, wie Zellstoffasern (wood pulp) oder Kunststofffasern enthalten. Denkbar ist weiterhin, den Saugkörper durch Anordnung von ein oder mehreren Schichten verschiedenen Materials, insbesondere aus Vliesstoff auszubilden. Der Saugkörper ist vorzugsweise integraler Bestandteil des Hauptteils und solchen Falls herstellerseitig unlösbar mit den weiteren Komponenten des Hauptteils verbunden. Solchen Falls ist der Saugkörper vorzugsweise unlösbar mit einem Topsheet und/oder mit einem Backsheet des Hauptteils verbunden.

Nach einer alternativen Ausführungsform ist der Saugkörper von den weiteren Komponenten des Hauptteils lösbar, insbesondere zum Zwecke der getrennten Entsorgung des gebrauchten mit Körperflüssigkeiten beladenen Saugkörpers. In diesem Fall, kann es vorteilhaft sein, den Saugkörper herstellerseitig getrennt von den übrigen Komponenten des Hauptteils bereitzustellen und Mittel vorzusehen, den Saugkörper mit den übrigen Komponenten des Hauptteils erst verbraucherseitig zu verbinden. Die übrigen Komponenten des Hauptteils sind solchen Falls vorzugsweise bereits herstellerseitig und ein Windelchassis bildend vorkonfektioniert.

Die hinteren Seitenabschnitte sind im einfachsten und bevorzugten Fall rechteckförmig ausgebildet, d.h. sie werden durch in der Querrichtung und in der Längsrichtung der Inkontinenzwegwerfwindel verlaufende Ränder begrenzt. Vorzugsweise wird zumindest der proximale Abschnitt der Erstreckung Q der Seitenabschnitte von in der Querrichtung verlaufenden Rändern begrenzt.

Vorteilhaft ist auch ein jeweiliger elastischer oder elastifizierter Bereich der hinteren Seitenabschnitte rechteckförmig ausgebildet, das heißt sie werden durch in der Querrichtung und in der Längsrichtung der Inkontinenzwegwerfwindel verlaufende Ränder begrenzt. Weiter vorzugsweise erstreckt sich ein jeweiliger elastischer oder elastifizierter Bereich über die volle Länge der hinteren Seitenabschnitte (in der Längsrichtung der Inkontinenzwegwerfwindel) .

Die Erstreckung eines jeweiligen elastischen oder elastifizierten Bereichs der hinteren Seitenabschnitte in der Querrichtung beträgt ungedehnt vorzugsweise 40 bis 120 mm, insbesondere 60 - 100 mm.

Die Erstreckung Q der eben ausgebreiteten jedoch nicht gedehnten hinteren Seitenabschnitte in der Querrichtung der Inkontinenzwegwerfwindel über den hinteren seitlichen Längsrand des Hauptteils hinaus beträgt vorzugsweise 130 bis 280 mm, insbesondere 170 bis 250 mm.

Die Erstreckung B der hinteren Seitenabschnitte in der Längsrichtung beträgt im Bereich der Anfügung an den Hauptteil 100 bis 200 mm, insbesondere 120 bis 170 mm.

Vorzugsweise ist eine Erstreckung QE eines jeweiligen elastischen oder elastifizierten Bereichs der hinteren Seitenabschnitte in der Querrichtung und eine maximale Erstreckung Q der hinteren Seitenabschnitte über den jeweiligen hinteren seitlichen Längsrand hinaus derart bemessen ist, dass das Verhältnis der Erstreckungen QE/Q zueinander 0,20 < QE/Q < 0,50, insbesondere 0,30 < QE/Q < 0,45 beträgt.

Es hat sich bei der erfindungsgemäßen T-förmigen Inkontinenzwegwerfwindel als besonders vorteilhaft erwiesen, wenn die Erstreckung Q der hinteren Seitenabschnitte in der Querrichtung über den jeweiligen hinteren seitlichen Längsrand hinaus und eine maximale Erstreckung B der hinteren Seitenabschnitte in der Längsrichtung derart bemessen ist, dass das Verhältnis der Erstreckungen Q/B zueinander 1,0 < Q/B < 2,0 beträgt. Dieses Verhältnis ist bei Gürtelwindeln um ein Vielfaches größer.

Es wird weiter vorgeschlagen, dass die beiden hinteren Seitenabschnitte in der Längsrichtung einen Abstand zu einem hinteren Querrand des Hauptteils von wenigstens 1 mm, insbesondere von wenigstens 5 mm, insbesondere von wenigstens 10 mm, insbesondere von wenigstens 15 mm, insbesondere von höchstens 50 mm aufweisen. Hierdurch ist gewährleistet, dass die beim Anlegen über die Verschlussmittel ausgeübten und dabei in den Rückbereich des Hauptteils eingeleiteten Querzugkräfte auf einen größeren Abschnitt des Hauptteils "verteilt" werden.

Weiter erweist sich als vorteilhaft, wenn eine in der Querrichtung erstreckte und das jeweilige Verschlussmittel auf der schrittzugewandten Seite tangierende gerade Linie den Saugkörper schneidet. Dies kann vorzugsweise dann realisiert werden, wenn die beiden hinteren Seitenabschnitte wie vorstehend erwähnt einen Abstand in der Längsrichtung zum hinteren Querrand des Hauptteils aufweisen. Insbesondere ist vorgesehen, dass eine in der Querrichtung erstreckte und die Seitenabschnitte in der Längsrichtung, im Bereich der Anfügung an den Hauptteil halbierende gerade Linie den Saugkörper schneidet. Es stabilisiert die Anlage des Saugkörpers und unterstützt einen korrekten Sitz der Windel.

Es erweist sich weiter als vorteilhaft, wenn jeder hintere Seitenabschnitt genau ein Verschlussmittel aufweist. Es handelt sich bei den Verschlussmitteln typischerweise um eine Lasche aus einem ein- oder mehrschichtigen Flachmaterial, welches ausgehend von einer in der Regel um einen distalen Längsrand des betrachteten Seitenabschnitts nach innen auf den Seitenabschnitt eingefalteten Konfiguration in eine nach außen ausgefaltete Betriebsstellung entfaltbar ist. In an sich bekannter und daher nicht näher zu beschreibender Weise ist ein jeweiliges Verschlussmittel mit klebenden und/oder mechanisch haftenden Bereichen, Schichten oder Elementen ausgestattet, wie zum Beispiel Haken/Schlaufen-Materialien. Sofern der Seitenabschnitt genau ein Verschlussmittel aufweist, so erweist es sich als vorteilhaft, wenn dieses Verschlussmittel in der Längsrichtung ungefähr mittig in einem distalen Bereich des Seitenabschnitts vorgesehen ist. Weiter erweist es sich als vorteilhaft, wenn das betreffende Verschlussmittel eine Erstreckung in der Längsrichtung zwischen 25 % und 75 % der Erstreckung B des Seitenabschnitts in der Längsrichtung beträgt. Ferner sind die jeweiligen Verschlussmittel vorzugsweise im ein- und ausgefalteten Zustand rechteckförmig ausgebildet. Sie sind in der herstellerseitigen nicht aktiven Konfiguration vorzugsweise nach innen auf sich selbst eingefaltet.

Hinsichtlich der Abmessungen des Hauptteils der Inkontinenzwegwerfwindel hat es sich als vorteilhaft erwiesen, wenn die Erstreckung des Hauptteils in der Querrichtung im Rückenbereich und/oder im Vorderbereich 250 bis 550 mm, insbesondere 300 bis 520 mm beträgt. Vorzugsweise weisen der Vorderbereich und der Rückenbereiche des Hauptteils dieselbe Quererstreckung (gemessen in mm) auf.

Die Erstreckung des Hauptteils in der Längsrichtung beträgt vorzugsweise 700 bis 1200 mm, insbesondere 800 bis 1100 mm.

Der Hauptteil kann im Schrittbereich mit einer Verengung in Querrichtung, mithin mit einer Beinöffnungskontur versehen sein. In einer alternativen Ausführungsform ist der Hauptteil rechteckförmig ausgebildet.

Die elastischen oder elastifizierten Bereiche der hinteren Seitenabschnitte können durch dem Fachmann an sich geläufige Mittel realisiert werden. So können beispielsweise Abschnitte per se elastischer Materialien, wie elastische Folien oder elastische Vliesstoffe, durch Fügeverfahren wie Kleben oder Thermoschweißen oder Ultraschallschweißen, an undehnbare Abschnitte der hinteren Seitenabschnitte angefügt werden.

Eine weitere Möglichkeit, eine bereichsweise Elastifizierung zu erreichen, besteht darin, dass die hinteren Seitenabschnitte bereichsweise vorzugsweise durch eine als "ring rolling" bekannt gewordene Technologie "aktiviert" werden. Diese Technologie ist beispielsweise in EP 0 650 714 A1 beschrieben. Durch "ring rolling" wird ein an sich nicht dehnbares Material, beispielsweise ein Vlies/Folien-Laminat durch übermäßige Auslenkung zwischen gegeneinander kämmenden Walzen überdehnt. In diesem überdehnten Zustand bringt das zuvor an sich nicht dehnbare Material des Laminates einer Längung im Wesentlichen keinen Widerstand entgegen. Durch Kombination mit einem elastisch dehnbaren Element innerhalb eines derartigen Laminates kann somit eine elastische Dehnbarkeit in dem entsprechend behandelten Bereich erreicht werden. Alternativ hierzu können bereichsweise elastifizierende Mittel wie elastische Folienabschnitte oder Fäden, insbesondere Lycra- oder Spandex-Fäden im vorgespannten Zustand mit den die Seitenabschnitte im Übrigen bildenden Flachmaterialien, insbesondere Vliesstoffmaterialien verbunden werden (stretch-bonding).

In der Gebrauchssituation werden die hinteren Seitenabschnitte in Überlappung mit der Außenseite des Vorderbereichs des Hauptteils gebracht, damit die im Bereich der jeweiligen freien Enden an beiden hinteren Seitenabschnitten vorgesehenen Verschlussmittel auf die Außenseite des Hauptteils der Windel geschlossen werden können. Hierzu sind die Verschlussmittel und mindestens ein Bereich der Außenseite des Hauptteils als Verschlusssystem ausgebildet. Insbesondere weisen die Verschlussmittel hierzu mechanische Verschlusselemente wie Kletthakenelemente auf, insbesondere auch in Kombination mit haftklebenden Bereichen, vermittels derer die Verschlussmittel lösbar haftend mit der Außenseite des Hauptteils in Eingriff bringbar sind. Hierzu hat es sich als vorteilhaft erwiesen, wenn die Außenseite des Hauptteils zumindest bereichsweise, vorzugsweise vollständig durch einen entsprechend ausgebildeten Vliesstoff gebildet ist. Alternativ ist möglich, ein separates Klettflauschelement auf der Außenseite des Hauptteils im Vorderbereich vorzusehen, welche als Landezone für die Verschlussmittel der Seitenabschnitte dient.

Obschon die hinteren Seitenabschnitte in der Querrichtung deutlich kürzer sind als bei Gürtelwindeln, erweist es sich als vorteilhaft, wenn die hinteren Seitenabschnitte herstellerseitig um wenigstens zwei in der Längsrichtung verlaufende Seitenabschnitt-Falzachsen auf sich selbst gefaltet sind und die so erhaltene gefaltete Konfiguration herstellerseitig vorzugsweise fixiert ist, etwa durch einzelne Fügestellen, insbesondere Klebe- oder Ultraschallschweißstellen, die gleichwohl zum Entfalten der Seitenabschnitte durch den Benutzer verhältnismäßig leicht manuell lösbar sind. Vorzugsweise in diesem Fall erweist sich ein einziges in der Längsrichtung ungefähr mittig an den Seitenabschnitten positioniertes Verschlussmittel als vorteilhaft, wobei dann die Fügestellen das eingefaltete Verschlussmittel nicht erfassen, sondern in der Längsrichtung außerhalb des Verschlussmittels angeordnet sind. Wenn die gegeneinander anliegenden Teilbereiche um das wenigstens eine nach innen eingeschlagene Verschlussmittel herum oder in der Längsrichtung oberhalb oder unterhalb des eingeschlagenen Verschlussmittels durch die vorgenannten Maßnahmen lösbar fixiert sind, so bildet das eingeschlagene Verschlussmittel einen gut ergreifbaren Anfassbereich zum Entfalten des jeweiligen Seitenabschnitts.

Weiter erweist sich als vorteilhaft, wenn die hinteren Seitenabschnitte herstellerseitig um wenigstens zwei in der Längsrichtung verlaufende Seitenabschnitt-Falzachsen auf sich selbst gefaltet sind und durch diese Seitenabschnitt-Falzachsen aufeinander gefaltete Teilbereiche der hinteren Seitenabschnitte definiert und begrenzt werden, und dass ein in der Querrichtung außen liegender Teilbereich im wesentlichen undehnbar ausgebildet ist. Hierdurch werden insbesondere die Ergreifbarkeit und die Entfaltbarkeit sowohl des Seitenabschnitts als auch des eingefalteten Verschlussmittels verbessert.

Weiter erweist sich als vorteilhaft, wenn ein an den außenliegenden Teilbereich nach innen anschließender Teilbereich ausgehend von der äußeren in der Längsrichtung verlaufenden Falzachse mit wenigstens 40 % seiner Fläche undehnbar ausgebildet ist. Diese wenigstens 40 % der Fläche werden also ausgehend von der außenliegenden Falzachse bestimmt, indem man hiervon ausgehend eine gedachte parallele Linie zur der Faltachse quasi scannend in der Querrichtung nach innen bewegt bis sie auf einen dehnbaren Bereich stößt. Es wird dann die gescannte Fläche ermittelt und ins Verhältnis zu der Gesamtaufsichtsfläche des Teilbereichs gesetzt. Hierdurch wird erreicht, dass der in der Querrichtung außen liegende Teilbereich und der daran nach innen anschließende Teilbereich über eine sehr große undehnbare Fläche (von wenigstens 40 % der Fläche des letztgenannten Teilbereichs), die demzufolge frei von elastischen oder elastifizierenden Elementen ist, flächenhaft aneinander anliegen.

Die Erstreckung U eines jeweiligen undehnbaren Bereichs des an den außenliegenden Teilbereich nach innen anschließenden Teilbereichs beträgt ausgehend von der äußeren in der Längsrichtung verlaufenden Falzachse in Querrichtung bis zum Beginn eines dehnbaren Bereichs vorzugsweise mindestens 15 mm, insbesondere mindestens 20 mm, weiter insbesondere mindestens 25 mm, weiter vorzugsweise mindestens 30 mm, vorzugsweise jedoch höchstens 100 mm, weiter vorzugsweise höchstens 70 mm.

Es wird weiter vorgeschlagen, dass eine jeweilige im ausgefalteten Zustand innenliegende, also dem hinteren seitlichen Längsrand des Hauptteils benachbarte Seitenabschnitt-Falzachse innerhalb des elastischen oder elastifizierten Bereichs des betreffenden Seitenabschnitts verläuft. Hierdurch kann die versteifende und an sich unerwünschte Wirkung einer jeden Faltung bei einem Flachmaterial reduziert werden.

Hingegen erweist es sich als vorteilhaft, wenn eine jeweilige im ausgefalteten Zustand in der Querrichtung weiter außen liegende Seitenabschnitt-Falzachse innerhalb des undehnbaren Bereichs der hinteren Seitenabschnitte verläuft. Dort kann nämlich die versteifende Wirkung der Falzachse durchaus erwünscht sein, da sich hierdurch die Einleitung der Zugkraft über die Verschlussmittel gleichmäßiger auf die Seitenabschnitte verteilt.

Es wird weiter vorgeschlagen, dass die hinteren Seitenabschnitte herstellerseitig um genau zwei in der Längsrichtung verlaufende Seitenabschnitt-Falzachsen auf sich selbst gefaltet sind, so dass genau drei Teilbereiche der Seitenabschnitte gebildet werden, und dass der mittlere Teilbereich ausgehend von der äußeren in der Längsrichtung verlaufenden Falzachse mit wenigstens 40 % seiner Fläche undehnbar ausgebildet ist.

Für die eingefaltete Konfiguration der hinteren Seitenabschnitte gilt vorzugsweise, dass eine Erstreckung A der auf sich selbst gefalteten hinteren Seitenabschnitte in der Querrichtung über den jeweiligen hinteren seitlichen Längsrand hinaus und eine Erstreckung B der auf sich selbst gefalteten hinteren Seitenabschnitte in der Längsrichtung derart bemessen ist, dass das Verhältnis der Erstreckungen A/B zueinander 0,5 < A/B <1 beträgt.

Vor der herstellerseitigen Verpackung der Inkontinenzwegwerfwindeln wird vorzugsweise der Hauptteil herstellerseitig mitsamt den auf sich selbst gefalteten hinteren Seitenabschnitten um eine erste und eine zweite jeweils in der Längsrichtung verlaufende Hauptteil-Falzachse nach innen auf sich selbst gefaltet, derart dass die beidseitigen hinteren Seitenabschnitte in Dickenrichtung, also orthogonal zu einer die Längsrichtung und die Querrichtung einschließenden Ebene, in wenigstens teilweiser Überlappung zueinander zu liegen kommen. Weiter vorzugsweise wird vor der herstellerseitigen Verpackung der Inkontinenzwegwerfwindeln der Hauptteil herstellerseitig mitsamt den auf sich selbst gefalteten hinteren Seitenabschnitten und vorzugsweise im Anschluss an die zuvor beschriebene Faltung um in der Längsrichtung verlaufende Hauptteil-Falzachsen zusätzlich um eine oder zwei in der Querrichtung verlaufende Hauptteil-Falzachsen nach innen auf sich selbst gefaltet.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Inkontinenzwegwerfwindel. In der Zeichnung zeigt:
Figur 1 eine Draufsicht auf eine erfindungsgemäße Inkontinenzwegwerfwindel im eben ausgebreiteten, jedoch nicht gedehnten Zustand;
Figur 2 eine Schnittansicht der Windel nach Figur eins mit Schnittebene II-II;
Figur 3 eine schematische Darstellung der Windel im angelegten Zustand;
Figuren 4a, b, c jeweils eine vergrößerte und teilweise Darstellung der Windel nach Figur 1 im Bereich eines hinteren Seitenabschnitts in eben ausgebreitetem, jedoch nicht gedehnten Zustand mit Bemaßungen bzw. mit Falzachsen;
Figur 5 eine vergrößerte Darstellung der Windel nach Figur 1 im Bereich eines hinteren Seitenabschnitts in auf sich selbst gefalteter Konfiguration;
Figur 6 eine Schnittansicht mit Schnittebene VI-VI Figur 5;
Figuren 7 und 8 eine Darstellung der Windel entsprechend Figur 4 mit Klemmen einer Vorrichtung zur Bestimmung der Dehnbarkeit.

Die Figuren zeigen, nicht maßstäblich, sondern schematisch eine erfindungsgemäße insgesamt mit dem Bezugszeichen 2 bezeichnete Inkontinenzwegwerfwindel in der sogenannten T-Form. Die Windel 2 umfasst einen insgesamt mit dem Bezugszeichen 4 bezeichneten Hauptteil mit einem Körperflüssigkeiten absorbierenden Saugkörper 6. Der Saugkörper 6 umfasst vorzugsweise Zellulosefasern und superabsorbierende Polymerpartikel (SAP). Der Saugkörper 6 ist zwischen zwei Flachmaterialien, und zwar einer flüssigkeitsdurchlässigen Deckschicht 60 (Topsheet) und einer flüssigkeitsundurchlässigen Rückschicht 62 (Backsheet) des Windelhauptteils 4 angeordnet.

Bei der Windel 2 ist eine Längsrichtung 8 und eine Querrichtung 10 unterscheidbar, wobei letztere im angelegten Zustand der Windel der Hüftumfangsrichtung des Benutzers entspricht. Der Hauptteil 4 umfasst einen Vorderbereich 12 mit vorderen seitlichen Längsrändern 14, einen Rückenbereich 16 mit hinteren seitlichen Längsrändern 18 und einen dazwischen angeordneten Schrittbereich 20. An einem jeweiligen Längsrand 15 des Schrittbereichs 20 angrenzend weist der Hauptteil 4 je einen elastifizierten Abschnitt 17, mithin einen elastifizierten Beinöffnungsabschnitt auf. Diese elastifizierten Beinöffnungsabschnitte sind gebildet durch zwischen Topsheet 60 und Backsheet 62 verlaufende und in vorgespanntem Zustand an Topsheet 60 und Backsheet 62 fixierte elastische Fäden, die bogenförmig gekrümmt, mithin mit einer Komponente in Längsrichtung 8 orientiert sind.

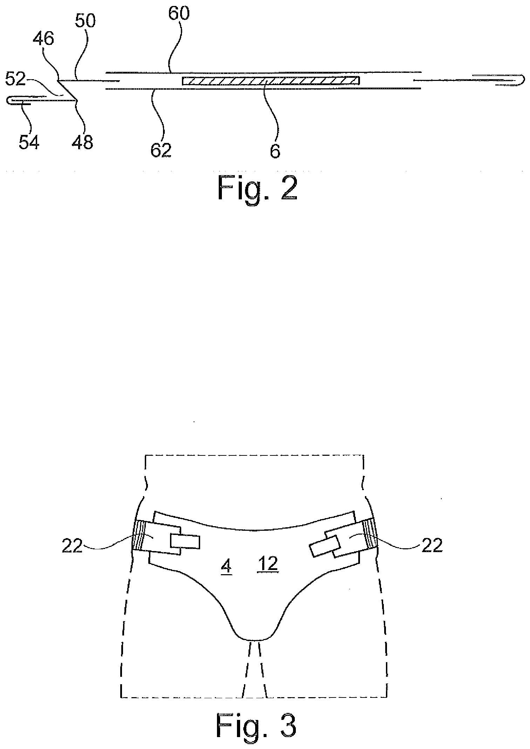

Bei der T-förmigen Windel 2 sind nur im Rückenbereich 16 des Hauptteils 4 in Querrichtung 10 seitlich über die hinteren seitlichen Längsränder 18 hinaus erstreckte hintere Seitenabschnitte 22 vorgesehen, die im Bereich der hinteren seitlichen Längsränder 18 in einem Überlappungsbereich 24 unlösbar an den Rückenbereich 16 des Hauptteils 4 angefügt sind. Die hinteren Seitenabschnitte 22 haben im Bereich ihres in Querrichtung 10 freien Endes 26 jeweils wenigstens ein Verschlussmittel 28. Das Verschlussmittel 28 ist in Form einer vorzugsweise rechteckigen Lasche ausgebildet und auf sich selbst eingefaltet. In der Gebrauchssituation lässt sich das Verschlussmittel öffnen, das heißt wieder auffalten, um die Inkontinenzwegwerfwindel 2 an einen Benutzer anzulegen, wobei die Seitenabschnitte 22 in Überlappung mit dem Vorderbereich 12 des Hauptteils 4 gebracht werden und die Verschlussmittel lösbar haftend auf der Außenseite des Vorderteils des Hauptteils festgelegt werden (schematisch dargestellt in Figur 3).

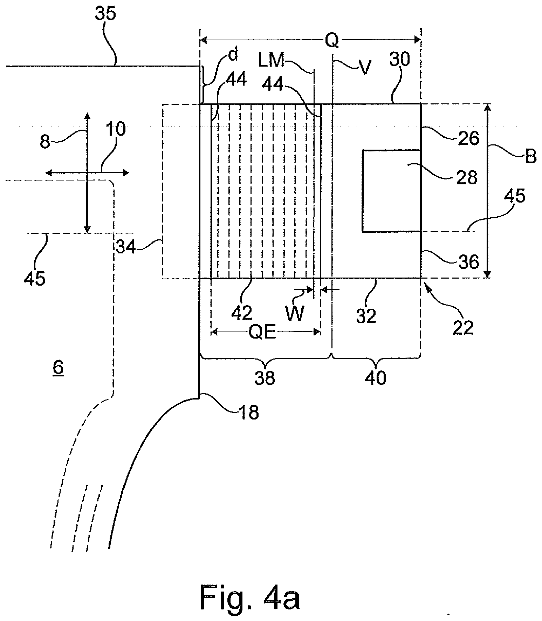

Die hinteren Seitenabschnitte 22 sind - wie am besten aus Figur 4a ersichtlich ist - vorzugsweise rechteckförmig ausgebildet, wobei sie durch in der Querrichtung 10 verlaufende Ränder 30, 32 und durch in der Längsrichtung 8 verlaufende Ränder 34, 36 begrenzt werden. In dem in Figur 4a dargestellten eben ausgefalteten oder ausgebreiteten jedoch nicht gedehnten Zustand haben die hinteren Seitenabschnitte 22 eine Erstreckung Q von 200 mm in der Querrichtung 10 über den hinteren seitlichen Längsrand 18 hinaus. Diese Erstreckung Q der hinteren Seitenabschnitte 22 in Querrichtung 10 außerhalb des Hauptteils 4 umfasst einen an den hinteren seitlichen Längsrand 18 anschließenden proximale Abschnitt 38 und einen frei endende distalen Abschnitt 40 der Seitenabschnitte 22.

Der proximale Abschnitt 38 wird im Rahmen der vorliegenden Erfindung als derjenige Abschnitt definiert, welcher sich in Querrichtung 10 ausgehend von dem hinteren seitlichen Längsrand 18 des Hauptteils 4 über eine Länge erstreckt, die 65% der Erstreckung Q eines hinteren Seitenabschnitts 20 beträgt. Entsprechend ist der distale Abschnitt 40 derjenige Abschnitt, welcher sich in Querrichtung 10 an den proximalen Abschnitt anschließend bis zum freien Ende 26 des Seitenabschnitts 22 erstreckt, mithin über eine Länge von 35 % der Erstreckung Q. In Figur 4a markiert eine virtuelle in der Längsrichtung 8 verlaufende Linie V die Grenze zwischen dem proximalem Abschnitt (38) und dem distalem Abschnitt (40).

Die hinteren Seitenabschnitte 22 haben in der Längsrichtung 8 einen Abstand d von vorzugsweise 5-50 mm von einem hinteren Querrand 35 der Windel. Die Erstreckung B der hinteren Seitenabschnitte in der Längsrichtung beträgt im dargestellten Fall 140 mm.

Die hinteren Seitenabschnitte 22 sind in Querrichtung 10 außerhalb des Hauptteils 4 elastisch dehnbar ausgebildet. Sie umfassen hierfür einen elastischen oder elastifizierten Bereich 42. Dieser elastische oder elastifizierte Bereich 42 der hinteren Seitenabschnitte 22 ist vollständig innerhalb des proximalen Abschnitts 38 der hinteren Seitenabschnitte 22 angeordnet. Er weist im beispielhaft dargestellten Fall in der Querrichtung 10 einen geringen Abstand von wenigen Millimetern von dem hinteren seitlichen Längsrand 18 auf. Der elastische oder elastifizierte Bereich 42 ist rechteckförmig ausgebildet und seine Erstreckung QE in der Querrichtung ist durch in der Längsrichtung 8 erstreckte Ränder 44 begrenzt, wobei die Ränder 44 sich über die gesamte Längserstreckung der hinteren Seitenabschnitte 22 erstrecken.

In der dargestellten Ausführungsform der Inkontinenzwegwerf-windel erstreckt sich der elastische oder elastifizierte Bereich 42 um das Maß W = 10 mm über eine Längsmittelachse LM eines jeweiligen hinteren Seitenabschnittes 22 hinaus, überfängt also die Längsmittelachse LM, wobei die Längsmittelachse LM im Rahmen der vorliegenden Erfindung diejenige virtuelle, in der Längsrichtung 8 verlaufende Linie ist, welche einen hinteren Seitenabschnitt 22 in zwei in der Querrichtung 10 gleich lang erstreckte Hälften teilt.

In dem distalen Abschnitt 40 der Erstreckung Q der hinteren Seitenabschnitte 22 sind die hinteren Seitenabschnitte 22 in der Querrichtung 10 undehnbar ausgebildet.

Typischerweise kann der jeweilige elastische oder elastifizierte Bereich 42 der hinteren Seitenabschnitte 22 durch Zwischenordnung eines elastisch dehnbaren oder eines elastifizierten Materials in die Seitenabschnitte 22 erreicht werden. Die Seitenabschnitte 22 sind dann durch in der Querrichtung 10 hintereinander angeordnete und miteinander gefügte Materialabschnitte verschiedener Dehnbarkeit ausgebildet. Alternativ hierzu können elastifizierende Mittel im vorgespannten Zustand mit Flachmaterialien der hinteren Seitenabschnitte 22 verbunden werden. Weiter alternativ ist es möglich, dass an sich undehnbare Flachmateriallaminate, wie beispielsweise Laminate umfassend undehnbare Vliesstoffe und daran flächenhaft angefügte elastische Folien der hinteren Seitenabschnitte 22 durch im Stand der Technik bekannte Maßnahmen, etwa durch das sogenannte "RingRolling", bereichsweise "aktiviert", das heißt elastisch dehnbar gemacht werden.

Die hinteren Seitenabschnitte 22 sind weiter derart am Rückenbereich 16 des Hauptteils 4 angeordnet, dass eine in der Querrichtung 10 erstreckte und das jeweilige Verschlussmittel 28 auf der schrittzugewandten Seite tangierende gerade Linie 45 den Saugkörper 6 schneidet.

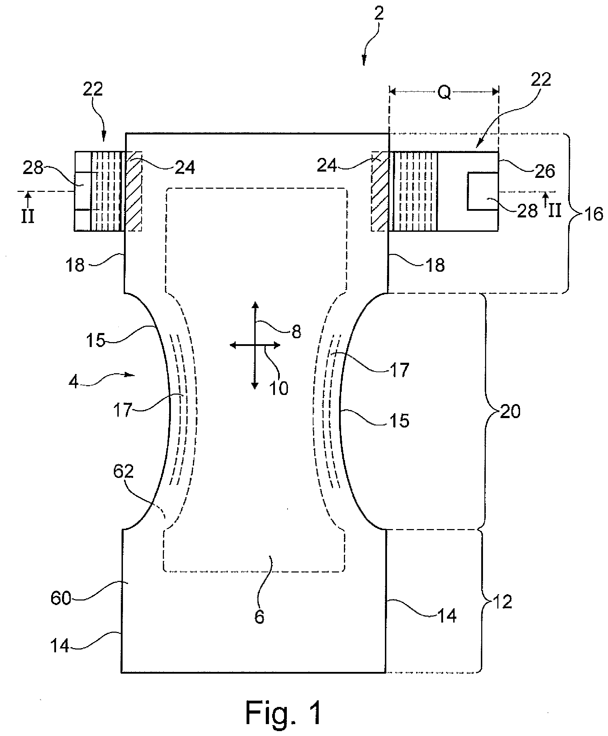

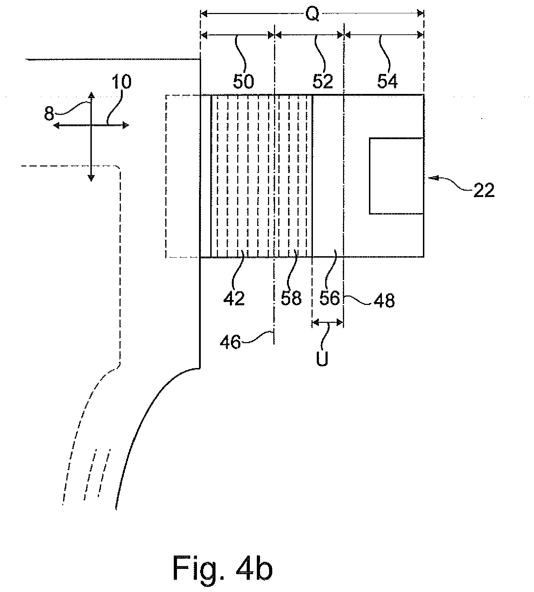

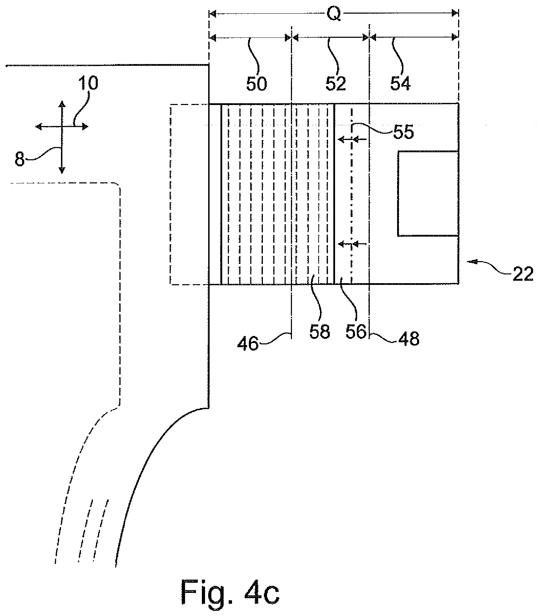

Wie in Figuren 1, 2 (jeweils links) und Figuren 4b, 5 und 6 dargestellt sind die hinteren Seitenabschnitte 22 herstellerseitig um wenigstens zwei in der Längsrichtung 8 verlaufende Seitenabschnitt-Falzachsen 46, 48 auf sich selbst gefaltet. Die Seitenabschnitt-Falzachsen 46, 48 definieren und begrenzen dabei aufeinander gefaltete Teilbereiche 50, 52, 54 der hinteren Seitenabschnitte 22 (Figur 4b). Man erkennt, dass die dem hinteren seiltichen Längsrand 18 benachbarte, also innenliegende Seitenabschnitt-Falzachse 46 innerhalb des elastischen oder elastifizierten Bereichs 42 verläuft, während die in der Querrichtung 10 weiter außen liegende Seitenabschnitt-Falzachse 48 außerhalb des elastischen oder elastifizierten Bereichs 42, also innerhalb eines undehnbaren Bereichs der Seitenabschnitte 22 verläuft. Der Teilbereich 52, welcher sich an den in der Querrichtung 10 außenliegenden Teilbereich 54 nach innen anschließt, ist ausgehend von der äußeren in der Längsrichtung 8 verlaufenden Falzachse 48 mit wenigstens 40 % seiner Fläche undehnbar ausgebildet. Zur Bestimmung dieser undehnbaren Fläche wird von der äußeren Falzachse 48 ausgehend und parallel hierzu eine gedachte Linie 55 quasi scannend in der Querrichtung 10 in Richtung auf den Hauptteil 4 nach innen bewegt (dies ist in Figur 4c durch Pfeile veranschaulicht) bis sie auf einen dehnbaren Bereich 58 stößt. Die so gescannte Fläche beträgt wenigstens 40 % der gesamten Aufsichtsfläche des Teilbereichs 52 im nicht gedehnten Zustand. Der Teilbereich 52 weist hier einen rechteckförmigen in der Querrichtung 10 äußeren undehnbaren Bereich 56 einer Quererstreckung U und einen rechteckförmigen in der Querrichtung 10 inneren elastisch dehnbaren Bereich 58 auf, die durch eine in der Längsrichtung 8 verlaufende gedachte Linie voneinander abgegrenzt sind. Dabei umfasst der rechteckförmige äußere undehnbare Bereich 56 - wie ausgeführt - wenigstens 40 % gesamten Aufsichtsfläche des Teilbereichs 52 im nicht gedehnten Zustand. Es ist auch denkbar, dass der sich an den außen liegenden Teilbereich 54 nach innen anschließende Teilbereich 52 über seine gesamte Erstreckung undehnbar ausgebildet ist.

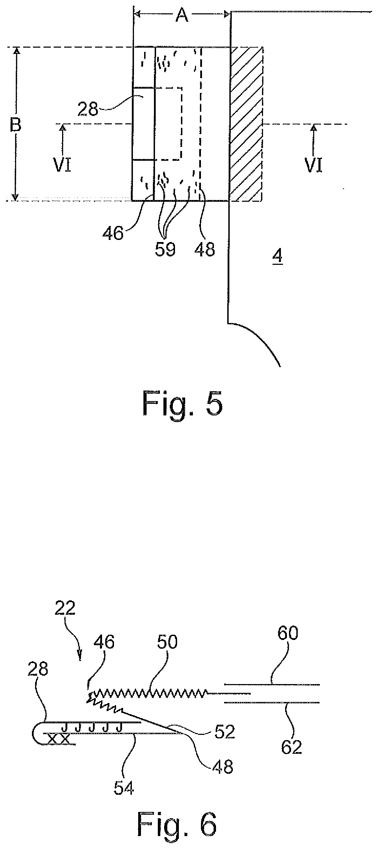

Die Figuren 5 und 6 zeigen schematisch die auf sich selbst gefaltete Konfiguration der hinteren Seitenabschnitte 22. Die gegeneinander gefalteten Teilbereiche 50, 52, 54 sind an in Figur 5 dargestellten Fügestellen 59 lösbar aneinander fixiert. Diese Fügestellen 59 sind durch eingangs beschriebene Maßnahmen ausgebildet. Es erweist sich als vorteilhaft, dass im Bereich der Überlappung der Teilbereiche 52, 54 zu einem wesentlichen Anteil undehnbare Bereiche miteinander lösbar gefügt sind. Man erkennt in Figur 6 auch in schematischer Darstellung die Anfügung des Verschlussmittels 28 beispielhaft an die körperabgewandte Außenseite des Seitenabschnitts 22 und die Umfaltung auf die körperzugewandte Seite des Seitenabschnitts 22. Ferner erkennt man die schematisch dargestellte Anfügung der hinteren Seitenabschnitte 22 beispielhaft zwischen zwei Flachmaterialien, beispielsweise eine flüssigkeitsdurchlässige Deckschicht 60 (Topsheet) und eine flüssigkeitsundurchlässige Rückschicht 62 (Backsheet) des Windelhauptteils 4.

Die Abmessung A der gefalteten Konfiguration der hinteren Seitenabschnitte 22 in der Querrichtung 10 außerhalb des Hauptteils 4 und die Abmessung B in der Längsrichtung 8 sind in Figur 5 dargestellt. Das Verhältnis A/B beträgt vorzugsweise 0,5 < A/B < 1. Das entsprechende Verhältnis Q/B der Seitenabschnitte 22 im eben ausgebreiteten jedoch nicht gedehnten Zustand beträgt vorzugsweise 1,0 < Q/B < 2,0 und ist in Figur 4a dargestellt. Das Verhältnis von QE, also der Erstreckung des elastischen oder elastifizierten Bereichs 42 in der Querrichtung, zu Q beträgt vorzugsweise 0,20 < QE/Q < 0,50, insbesondere 0,30 < QE/Q < 0,45.

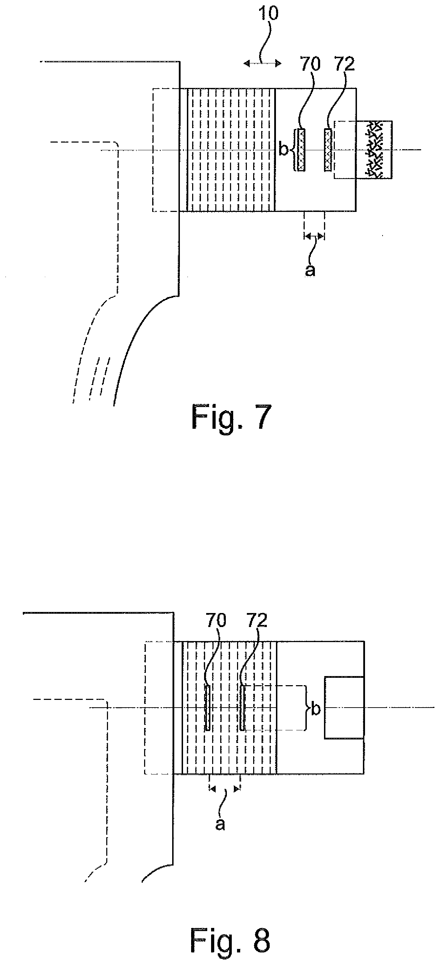

Sofern es sich im Einzelfall als nicht offensichtlich erweisen sollte, ob ein Seitenabschnittsbereich elastisch dehnbar oder undehnbar ist, wird zur quantitativen Abgrenzung der Begriffe undehnbar, dehnbar bzw. elastisch dehnbar das nachfolgende Testverfahren angegeben:
Die Dehnbarkeit kann hierbei direkt am Seitenabschnitt 22 der Windel bestimmt werden. Hierfür wird ein betreffender Bereich eines Seitenabschnitts 22 zwischen zwei Klemmbacken 70, 72 von definierter, gleicher Klemmbackenbreite b von 50 mm eingespannt, wobei der Klemmbackenabstand a 30 mm beträgt (Figuren 7 und 8). Dabei erstreckt sich die Klemmbackenbreite b in der Längsrichtung 8 und der Klemmbackenabstand a in der Querrichtung 10, wobei sich der Seitenabschnitt 22 in eben ausgebreitetem jedoch nicht gedehnten Zustand befindet. Sofern der zu erfassende Bereich in der Querrichtung 10 eine Erstreckung von weniger als 30 mm haben sollte, wird der Klemmbackenabstand a entsprechend geringer gewählt. Die Prüfung erfolgt ausgehend von einer Vorkraft von 0,2 N zwischen den Klemmbacken 70, 72. Die Klemmbacken 70, 72 werden hiervon ausgehend mit einer konstanten Geschwindigkeit von 100 mm/min bis zum Erreichen einer Kraft von 15 N in der Querrichtung 10 voneinander weg bewegt und im Wesentlichen unmittelbar nach Erreichen der Kraft von 15 N wieder aufeinander zu bewegt, und zwar wiederum mit einer konstanten Geschwindigkeit von 100 mm/min bis zum Erreichen des Werts der Vorkraft von 0,2 N. Es wird zum einen der initiale Klemmbackenabstand L0 in mm bei Erreichen der Vorkraft von 0,2 N notiert. Des Weiteren wird der Klemmbackenabstand L1 in mm bei Erreichen der Kraft von 15 N notiert (vorzugsweise erfolgt dies jeweils automatisiert über die Auswerteeinheit der Prüfvorrichtung). Ferner wird am Ende der Prüfung der verbleibende Klemmbackenabstand L2 in mm nach Zurücknahme der Kraft auf den Wert der Vorkraft von 0,2 N notiert.

Im Falle der Prüfung der Dehnbarkeit im Bereich des distalen Abschnitts 40 der hinteren Seitenabschnitte 22 werden die Klemmbacken 70, 72 vorzugsweise derart positioniert, dass sie außerhalb von Verschlussmitteln angeordnet sind. Hierfür kann wie in Figur 7 dargestellt das Verschlussmittel nach außen ausgefaltet werden. Die Prüfung der von dem Verschlussmittel überfangenen Fläche ist nicht erforderlich, da die Seitenabschnitte dort aufgrund der Verschlussmittel in der Regel ohnehin undehnbar sind.

Im Falle der Prüfung der elastischen Dehnbarkeit innerhalb des proximalen Abschnitts 38 der Seitenabschnitte 22 werden die Klemmbacken 70, 72 vorzugsweise in der Längsrichtung 8 mittig bezüglich der Längserstreckung der Seitenabschnitte 22 angeordnet, so wie dies in Figur 8 dargestellt ist. Es wird dann die vorstehende Prüfung ausgeführt. Im Anschluss daran werden die Klemmbacken 70, 72 in der Längsrichtung 8 versetzt (in der Figur 8 nach oben bzw. unten versetzt), so dass in der Längsrichtung 8 angrenzende Bereiche des Seitenabschnitts erfasst werden können und der Seitenabschnitt somit über seine gesamte Erstreckung in der Längsrichtung 8 getestet werden kann.

Für die Auswertung wird unter dem Begriff der Dehnung das Verhältnis zwischen einer Zunahme des Klemmbackenabstands bei Erreichen von 15 N und dem Klemmbackenabstand bei der Vorkraft von 0,2 N verstanden, also gedehnter Klemmbackenabstand bei 15 N L1 in mm minus L0 in mm geteilt durch L0 in mm, also Dehnung [%] = (L1 - L0) / L0.

Für die Ermittlung der bleibenden Dehnung (permanent set) wird das Verhältnis der Zunahme des Klemmbackenabstands nach Zurücknahme der Kraft auf den Wert der Vorkraft von 0,2 N gegenüber dem initialen Klemmbackenabstand (L2 - L0) und des initialen Klemmbackenabstands L0 verstanden, also bleibende Dehnung [%] = (L2 - L0) / L0.

Im Rahmen der vorliegenden Erfindung wird der distale Abschnitt 40 oder ein beliebiger Bereich eines jeweiligen hinteren Seitenabschnitts 22 als undehnbar angesehen, wenn bei Ausführung des vorstehenden Tests jeder in der Längsrichtung 8 50 mm und in der Querrichtung 10 30 mm erstreckte Bereich des distalen Abschnitts 40 bei kurzzeitiger Einwirkung einer Kraft von 15 N eine Dehnung von weniger als 50 % zulässt.

Im Rahmen der vorliegenden Erfindung wird der proximale Abschnitt 38 oder ein Bereich 42 des proximalen Abschnitts 38 oder ein beliebiger Bereich eines jeweiligen hinteren Seitenabschnitts 22 als elastisch dehnbar angesehen, wenn bei Ausführung des vorstehenden Tests der von den Klemmbacken erfasste Bereich bei 15 N eine Dehnung von wenigstens 60 % zulässt und nach Zurücknahme der Kraft auf den Wert der Vorkraft von 0,2 N eine bleibende Dehnung (permanent set) von höchstens 15 % verbleibt, und zwar auch bei Ausführung des Tests mit in der Längsrichtung 8 versetzten Klemmbacken. Es wird also der Seitenabschnitt 22 über seine gesamte Erstreckung in der Längsrichtung 8 getestet.

Im Falle, dass das zwischen den Klemmbacken eingespannte Material eines Seitenabschnitts bei der Ausführung des vorstehenden Tests bricht, bevor die maximale Kraft von 15 N erreicht ist und die Bruchkraft somit weniger als 15N/50mm beträgt, wird das Material als nicht zur Ausführung der Erfindung geeignet angesehen.

## Patentansprüche

1. Inkontinenzwegwerfwindel (2), mit einem einen Saugkörper (6) aufweisenden Hauptteil (4) mit einer Längsrichtung (8) und einer Querrichtung (10), umfassend einen Vorderbereich (12) mit vorderen seitlichen Längsrändern (14), einen Rückenbereich (16) mit hinteren seitlichen Längsrändern (18) und einen dazwischen angeordneten, zwischen den Beinen eines Benutzers zu liegen kommenden Schrittbereich (20), und mit beidseits an den Rückenbereich (16) angefügten hinteren Seitenabschnitten (22), welche sich in der Querrichtung (10) der Inkontinenzwegwerfwindel (2) über die seitlichen hinteren Längsränder (18) des Hauptteils (4) hinaus erstrecken und im Bereich ihres in der Querrichtung (10) freien Endes (26) jeweils wenigstens ein Verschlussmittel (28) tragen, wohingegen an den Vorderbereich (14) keine Seitenabschnitte (22) angefügt sind, sondern die vorderen seitlichen Längsränder (14) des Hauptteils (4) einen frei endenden Längsrand der Windel bilden, wobei die hinteren Seitenabschnitte (22) zum Anlegen und Schließen der Inkontinenzwegwerfwindel (2) an einen Benutzer jeweils entlang einer Umfangsrichtung um den Körper des Benutzers herumlegbar und in überlappende Anordnung mit einer Außenseite des Vorderbereichs (12) bringbar sind, an der sie dann über das jeweilige Verschlussmittel (28) jeweils lösbar anhaftbar sind, wobei die hinteren Seitenabschnitte (22) in eben ausgebreitetem jedoch nicht gedehnten Zustand eine Erstreckung Q in der Querrichtung (10) über den jeweiligen hinteren seitlichen Längsrand (18) hinaus aufweisen, wobei eine Längsmittelachse (LM) der hinteren Seitenabschnitte (22) die Erstreckung Q halbiert, und wobei die hinteren Seitenabschnitte (22) innerhalb dieser Erstreckung Q in der Querrichtung (10) elastisch dehnbar sind und hierfür einen in der Querrichtung (10) und in der Längsrichtung (8) erstreckten elastischen oder elastifizierten Bereich (42) aufweisen, wobei diese Erstreckung Q der hinteren Seitenabschnitte (22) in der Querrichtung (10) einen an den hinteren seitlichen Längsrand (18) anschließenden proximale Abschnitt (38) und einen an den proximalen Abschnitt (38) anschließenden, frei endenden distalen Abschnitt (40) umfasst, und wobei der proximale Abschnitt (38) sich in Querrichtung (10) ausgehend von dem hinteren seitlichen Längsrand (18) über eine Länge erstreckt, die 65% der Erstreckung Q eines hinteren Seitenabschnitts (22) beträgt, und wobei der Hauptteil (4) in dem Schrittbereich (20) beidseitig an einen jeweiligen Längsrand (15) des Schrittbereichs angrenzend je einen in Längsrichtung erstreckten elastischen oder elastifizierten Abschnitt (17) aufweist, **dadurch gekennzeichnet, dass** der jeweilige elastische oder elastifizierte Bereich (42) der hinteren Seitenabschnitte (22) vollständig innerhalb des proximalen Abschnitts (38) angeordnet ist und an den zugeordneten hinteren seitlichen Längsrand (18) heranreicht oder einen Abstand von dem hinteren seitlichen Längsrand (18) in der Querrichtung (10) von höchstens 30 mm aufweist, und dass die jeweiligen hinteren Seitenabschnitte (22) in dem gesamten distalen Abschnitt (40) im wesentlichen undehnbar ausgebildet sind.

2. Inkontinenzwegwerfwindel nach Anspruch 1, **dadurch gekennzeichnet, dass** der jeweilige elastische oder elastifizierte Bereich (42) der hinteren Seitenabschnitte (22) an den zugeordneten hinteren seitlichen Längsrand (18) heranreicht, jedoch ohne den hinteren seitlichen Längsrand zu überlappen.

3. Inkontinenzwegwerfwindel nach Anspruch 1 oder 2, **dadurch gekennzeichnet, dass** die beiden hinteren Seitenabschnitte (22) mit einem undehnbaren den Hauptteil (4) überlappenden Bereich (24) an den Hauptteil (4) unlösbar angefügt sind.

4. Inkontinenzwegwerfwindel nach Anspruch 1, 2 oder 3, **dadurch gekennzeichnet, dass** der jeweilige elastische oder elastifizierte Bereich (42) in der Querrichtung (10) um wenigstens 70 %, insbesondere um wenigstens 80 %, weiter insbesondere um wenigstens 90 % dehnbar ist.

5. Inkontinenzwegwerfwindel nach einem oder mehreren der vorstehenden Ansprüche, wobei der elastische oder elastifizierte Bereich (42) die Längsmittelachse (LM) eines jeweiligen hinteren Seitenabschnittes (22) überfängt.

6. Inkontinenzwegwerfwindel nach einem oder mehreren der vorstehenden Ansprüche, **dadurch gekennzeichnet, dass** die Erstreckung Q der hinteren Seitenabschnitte (22) in der Querrichtung (10) über den jeweiligen hinteren seitlichen Längsrand (18) hinaus und eine maximale Erstreckung B der hinteren Seitenabschnitte (22) in der Längsrichtung (8) derart bemessen ist, dass das Verhältnis der Erstreckungen (Q/B) zueinander 1,0 < Q/B < 2,0 beträgt.

7. Inkontinenzwegwerfwindel nach einem oder mehreren der vorstehenden Ansprüche, **dadurch gekennzeichnet, dass** die beiden hinteren Seitenabschnitte (22) in der Längsrichtung (8) einen Abstand (d) zu einem hinteren Querrand (35) des Hauptteils (4) von wenigstens 1 mm, insbesondere von wenigstens 5 mm, insbesondere von wenigstens 10 mm, insbesondere von wenigstens 15 mm, insbesondere von höchstens 50 mm aufweisen.

8. Inkontinenzwegwerfwindel nach einem oder mehreren der vorstehenden Ansprüche, **dadurch gekennzeichnet, dass** eine in der Querrichtung erstreckte und das jeweilige Verschlussmittel auf der schrittzugewandten Seite tangierende gerade Linie (45) den Saugkörper (6) schneidet.

9. Inkontinenzwegwerfwindel nach einem oder mehreren der vorstehenden Ansprüche, **dadurch gekennzeichnet, dass** jeder hintere Seitenabschnitt (22) genau ein Verschlussmittel (28) aufweist.

10. Inkontinenzwegwerfwindel nach einem oder mehreren der vorstehenden Ansprüche, **dadurch gekennzeichnet, dass** die hinteren Seitenabschnitte (22) herstellerseitig um wenigstens zwei in der Längsrichtung (8) verlaufende Seitenabschnitt-Falzachsen (46, 48) auf sich selbst gefaltet sind und durch diese Seitenabschnitt-Falzachsen (46, 48) aufeinander gefaltete Teilbereiche (50, 52, 54) der hinteren Seitenabschnitte (22) definiert und begrenzt werden, und dass ein in der Querrichtung (10) außenliegender Teilbereich (54) im wesentlichen undehnbar ausgebildet ist.

11. Inkontinenzwegwerfwindel nach Anspruch 10, **dadurch gekennzeichnet, dass** ein an den außenliegenden Teilbereich (54) nach innen anschließender Teilbereich (52) ausgehend von der äußeren in der Längsrichtung verlaufenden Falzachse (48) mit wenigstens 40 % seiner Fläche undehnbar ausgebildet ist.

12. Inkontinenzwegwerfwindel nach Anspruch 10 oder 11, **dadurch gekennzeichnet, dass** eine jeweilige im ausgefalteten Zustand innenliegende, also dem hinteren seitlichen Längsrand (18) des Hauptteils (4) benachbarte Seitenabschnitt-Falzachse (46) innerhalb des elastischen oder elastifizierten Bereichs (42) des betreffenden Seitenabschnitts (22) verläuft.

13. Inkontinenzwegwerfwindel nach Anspruch 10, 11 oder 12, **dadurch gekennzeichnet, dass** eine jeweilige im ausgefalteten Zustand in der Querrichtung (10) weiter außen liegende Seitenabschnitt-Falzachse (48) innerhalb eines undehnbaren Bereichs der hinteren Seitenabschnitte (22) verläuft.

14. Inkontinenzwegwerfwindel nach einem oder mehreren der vorstehenden Ansprüche, **dadurch gekennzeichnet, dass** die hinteren Seitenabschnitte (22) herstellerseitig um genau zwei in der Längsrichtung (8) verlaufende Seitenabschnitt-Falzachsen (46, 48) auf sich selbst gefaltet sind, so dass genau drei Teilbereiche (50, 52, 54) der Seitenabschnitte (22) gebildet werden, und dass der mittlere Teilbereich (52) ausgehend von der äußeren in der Längsrichtung (8) verlaufenden Falzachse (48) mit wenigstens 40 % seiner Fläche undehnbar ausgebildet ist.

15. Inkontinenzwegwerfwindel nach einem oder mehreren der Ansprüche 10-14, **dadurch gekennzeichnet, dass** eine Erstreckung (A) der auf sich selbst gefalteten hinteren Seitenabschnitte (22) in der Querrichtung (10) über den jeweiligen hinteren seitlichen Längsrand (18) hinaus und eine Erstreckung B der auf sich selbst gefalteten hinteren Seitenabschnitte (22) in der Längsrichtung (8) derart bemessen ist, dass das Verhältnis der Erstreckungen (A/B) zueinander 0,5 < A/B < 1 beträgt.

16. Inkontinenzwegwerfwindel nach einem oder mehreren der Ansprüche 10-15, **dadurch gekennzeichnet, dass** der Hauptteil (4) herstellerseitig mitsamt den auf sich selbst gefalteten hinteren Seitenabschnitten (22) um eine erste und eine zweite jeweils in der Längsrichtung (8) verlaufende Hauptteil-Falzachse nach innen auf sich selbst gefaltet ist, derart dass die beidseitigen hinteren Seitenabschnitte (22) in Dickenrichtung in wenigstens teilweiser Überlappung zueinander zu liegen kommen.

17. Inkontinenzwegwerfwindel nach einem oder mehreren der vorstehenden Ansprüche, **dadurch gekennzeichnet, dass** der Saugkörper superabsorbierendes Polymermaterial (SAP) enthält, insbesondere zu 5-100 Gewichtsprozent, vorzugsweise zu 10-95 Gewichtsprozent, weiter vorzugsweise zu 15-90 Gewichtsprozent und ganz besonders bevorzugt zu 20-80 Gewichtsprozent.

## Claims

1. Disposable incontinence nappy (2), having a main part (4) having an absorbent element (6) and a longitudinal direction (8) and a transverse direction (10), comprising a front region (12) having forward lateral longitudinal peripheries (14), a back region (16) having rearward lateral longitudinal peripheries (18), and disposed therebetween a crotch region (20) that comes to lie between the legs of a user, and having rearward side portions (22) which are joined to the back region (16) on both sides and which in the transverse direction (10) of the disposable incontinence nappy (2) extend beyond the rearward lateral longitudinal peripheries (18) of the main part (4) and in the region of the free end (26) of said rearward side portions (22) in the transverse direction (10) carry in each case at least one closure means (28), whereas no side portions (22) are joined to the front region (14), but rather the forward lateral longitudinal peripheries (14) of the main part (4) form a free-ending longitudinal periphery of the nappy, wherein the rearward side portions (22) for placing and closing the disposable incontinence nappy (2) on a user are in each case capable of being placed in a circumferential direction around the body of the user and are capable of being brought to an arrangement that overlaps with an external side of the front region (12) on which said rearward side portions (22) by way of the respective closure means (28) are in each case capable of adhering in a releasable manner, wherein the rearward side portions (22) in a planar spread-out but not elongated state have an extent Q in the transverse direction (10) beyond the respective rearward lateral longitudinal periphery (18), wherein a longitudinal central axis (LM) of the rearward side portions (22) bisects the extent Q, and wherein the rearward side portions (22) within this extent Q are elastically elongatable in the transverse direction (10) and to this end have an elastic or elasticized region (42) that extends in the transverse direction (10) and in the longitudinal direction (8), wherein this extent Q of the rearward side portions (22) in the transverse direction (10) comprises a proximal portion (38) that adjoins the rearward lateral longitudinal periphery (18), and a free-ending distal portion (40) that adjoins the proximal portion (38), and wherein the proximal portion (38), proceeding from the rearward lateral longitudinal periphery (18), in the transverse direction (10) extends across a length which is 65% of the extent Q of a rearward side portion (22), and wherein the main part (4) in the crotch region (20) on both sides, so as to be adjacent to a respective longitudinal periphery (15) of the crotch region, has in each case one elastic or elasticized portion (17) that extends in the longitudinal direction, **characterized in that** the respective elastic or elasticized region (42) of the rearward side portions (22) is disposed completely within the proximal portion (38) and reaches up to the assigned rearward lateral longitudinal periphery (18), or in the transverse direction (10) has a spacing of at most 30 mm from the rearward lateral longitudinal periphery (18), and **in that** the respective rearward side portions (22) in the entire distal portion (40) are configured so as to be substantially non-elongatable.

2. Disposable incontinence nappy according to Claim 1, **characterized in that** the respective elastic or elasticized region (42) of the rearward side portions (22) reaches up to the assigned rearward lateral longitudinal periphery (18), without however overlapping the rearward lateral longitudinal periphery.

3. Disposable incontinence nappy according to Claim 1 or 2, **characterized in that** the two rearward side portions (22), conjointly with a non-elongatable region (24) that overlaps the main part (4), are non-releasably joined to the main part (4).

4. Disposable incontinence nappy according to Claim 1, 2, or 3, **characterized in that** the respective elastic or elasticized region (42) in the transverse direction (10) is elongatable by at least 70%, in particular by at least 80%, furthermore particularly by at least 90%.

5. Disposable incontinence nappy according to one or a plurality of the preceding claims, wherein the elastic or elasticized region (42) engages across the longitudinal central axis (LM) of a respective rearward side portion (22).

6. Disposable incontinence nappy according to one or a plurality of the preceding claims, **characterized in that** the extent Q in the transverse direction (10) of the rearward side portions (22) beyond the respective rearward lateral longitudinal periphery (18), and a maximum extent B in the longitudinal direction (8) of the rearward side portions (22), are dimensioned such that the mutual ratio of the extents (Q/B) is 1.0 < Q/B < 2.0.

7. Disposable incontinence nappy according to one or a plurality of the preceding claims, **characterized in that** the two rearward side portions (22) in the longitudinal direction (8) have a spacing (d) from a rearward transverse periphery (35) of the main part (4) of at least 1 mm, in particular of at least 5 mm, in particular of at least 10 mm, in particular of at least 15 mm, in particular of at most 50 mm.

8. Disposable incontinence nappy according to one or a plurality of the preceding claims, **characterized in that** a straight line (45) that extends in the transverse direction and is tangent to the respective closure means on the crotch-facing side intersects the absorbent element (6).

9. Disposable incontinence nappy according to one or a plurality of the preceding claims, **characterized in that** each rearward side portion (22) has exactly one closure means (28).

10. Disposable incontinence nappy according to one or a plurality of the preceding claims, **characterized in that** the rearward side portions (22) ex works are folded onto themselves about at least two side portion folding axes (46, 48) that run in the longitudinal direction (8) and on account of these side portion folding axes (46, 48) part-regions (50, 52, 54), folded on top of one another, of the rearward side portions (22) are defined and delimited, and **in that** a part-region (54) that in the transverse direction (10) is outboard is configured so as to be substantially non-elongatable.

11. Disposable incontinence nappy according to Claim 10, **characterized in that** a part-region (52) that inwardly adjoins the outboard part-region (54), proceeding from the outer folding axis (48) that runs in the longitudinal direction, by way of at least 40% of the area of said part-region (52) is configured so as to be non-elongatable.

12. Disposable incontinence nappy according to Claim 10 or 11, **characterized in that** a respective side portion folding axis (46) that in the unfolded state is inboard, thus adjacent to the rearward lateral longitudinal periphery (18) of the main part (4), runs within the elastic or elasticized region (42) of the respective side portion (22).

13. Disposable incontinence nappy according to Claim 10, 11, or 12, **characterized in that** a respective side portion folding axis (48) that in the unfolded state is further outboard in the transverse direction (10) runs within a non-elongatable region of the rearward side portions (22).

14. Disposable incontinence nappy according to one or a plurality of the preceding claims, **characterized in that** the rearward side portions (22) ex works are folded onto themselves about exactly two side portion folding axes (46, 48) that run in the longitudinal direction (8) such that exactly three part-regions (50, 52, 54) of the side portions (22) are formed, and **in that** the central part-region (52), proceeding from the outer folding axis (48) that runs in the longitudinal direction (8), by way of at least 40% of the area thereof is configured so as to be non-elongatable.

15. Disposable incontinence nappy according to one or a plurality of Claims 10 to 14, **characterized in that** an extent (A) in the transverse direction (10) of the rearward side portions (22) that are folded onto themselves beyond the respective rearward lateral longitudinal periphery (18), and an extent B in the longitudinal direction (8) of the rearward side portions (22) that are folded onto themselves, are dimensioned such that the mutual ratio of the extents (A/B) is 0.5 < A/B < 1.

16. Disposable incontinence nappy according to one or a plurality of Claims 10 to 15, **characterized in that** the main part (4), together with the rearward side portions (22) that are folded onto themselves, is ex works folded inward onto itself about a first and a second main part folding axis that in each case run in the longitudinal direction (8) in such a manner that the rearward side portions (22) on both sides come to lie so as to at least partially overlap one another in the direction of thickness.

17. Disposable incontinence nappy according to one or a plurality of the preceding claims, **characterized in that** the absorbent element, in particular to an extent of 5 to 100 percent by weight, preferably of 10 to 95 percent by weight, furthermore preferably of 15 to 90 percent by weight, and particularly preferably of 20 to 80 percent by weight, contains super-absorbent polymer material (SAP).

## Revendications

1. Couche jetable pour l'incontinence (2), présentant une partie principale (4) comprenant un corps absorbant (6), présentant une direction longitudinale (8) et une direction transversale (10), comportant une zone avant (12) présentant des bords longitudinaux latéraux avant (14), une zone dorsale (16) présentant des bords longitudinaux latéraux arrière (18), et une zone d'entrejambe (20) agencée entre elles, venant se situer entre les jambes d'un utilisateur, et présentant des sections latérales arrière (22) assemblées des deux côtés à la zone dorsale (16), qui s'étendent dans la direction transversale (10) de la couche jetable pour l'incontinence (2) au-delà des bords longitudinaux latéraux arrière (18) de la partie principale (4) et qui portent chacun au moins un moyen de fermeture (28) dans la zone de leur extrémité libre (26) dans la direction transversale (10), tandis qu'aucune section latérale (22) n'est assemblée à la zone avant (14), mais les bords longitudinaux latéraux avant (14) de la partie principale (4) forment un bord longitudinal se terminant librement de la couche, les sections latérales arrière (22), pour la mise en place et la fermeture de la couche jetable pour l'incontinence (2) sur un utilisateur, pouvant à chaque fois être placées, le long d'une direction périphérique, autour du corps de l'utilisateur et être amenées dans un agencement de chevauchement avec un côté extérieur de la zone avant (12), à laquelle elles peuvent ensuite adhérer chacune de manière amovible par l'intermédiaire du moyen de fermeture respectif (28), les sections latérales arrière (22) présentant, dans un état déployé à plat mais non étiré, une étendue Q dans la direction transversale (10) au-delà du bord longitudinal latéral arrière respectif (18), un axe médian longitudinal (LM) des sections latérales arrière (22) coupant en deux l'étendue Q, et les sections latérales arrière (22) étant élastiquement extensibles à l'intérieur de cette étendue Q dans la direction transversale (10) et comprenant à cette fin une zone élastique ou rendue élastique (42), s'étendant dans la direction transversale (10) et dans la direction longitudinale (8), cette étendue Q des sections latérales arrière (22) dans la direction transversale (10) comportant une section proximale (38) se raccordant au bord longitudinal latéral arrière (18) et une section distale (40) se terminant librement, se raccordant à la section proximale (38), et la section proximale (38) s'étendant dans la direction transversale (10), à partir du bord longitudinal latéral arrière (18), sur une longueur qui représente 65 % de l'étendue Q d'une section latérale arrière (22), et la partie principale (4) comprenant dans la zone d'entrejambe (20), des deux côtés en position voisine d'un bord longitudinal respectif (15) de la zone d'entrejambe, à chaque fois une section élastique ou rendue élastique (17) s'étendant dans la direction longitudinale, **caractérisée en ce que** la zone élastique ou rendue élastique respective (42) des sections latérales arrière (22) est agencée entièrement à l'intérieur de la section proximale (38) et s'étend jusqu'au bord longitudinal latéral arrière associé (18) ou présente une distance par rapport au bord longitudinal latéral arrière (18) dans la direction transversale (10) d'au plus 30 mm, et **en ce que** les sections latérales arrière respectives (22) sont configurées sous forme essentiellement inextensible dans la totalité de la section distale (40).

2. Couche jetable pour l'incontinence selon la revendication 1, **caractérisée en ce que** la zone élastique ou rendue élastique respective (42) des sections latérales arrière (22) s'étend jusqu'au bord longitudinal latéral arrière associé (18), toutefois sans chevaucher le bord longitudinal latéral arrière.

3. Couche jetable pour l'incontinence selon la revendication 1 ou 2, **caractérisée en ce que** les deux sections latérales arrière (22) sont assemblées de manière non amovible à la partie principale (4) avec une zone inextensible (24) chevauchant la partie principale (4).

4. Couche jetable pour l'incontinence selon la revendication 1, 2 ou 3, **caractérisée en ce que** la zone élastique ou rendue élastique respective (42) est extensible dans la direction transversale (10) d'au moins 70 %, particulièrement d'au moins 80 %, plus particulièrement d'au moins 90 %.

5. Couche jetable pour l'incontinence selon une ou plusieurs des revendications précédentes, **caractérisée en ce que** la zone élastique ou rendue élastique (42) recouvre l'axe médian longitudinal (LM) d'une section latérale arrière respective (22).

6. Couche jetable pour l'incontinence selon une ou plusieurs des revendications précédentes, **caractérisée en ce que** l'étendue Q des sections latérales arrière (22) dans la direction transversale (10) au-delà du bord longitudinal latéral arrière respectif (18) et une étendue maximale B des sections latérales arrière (22) dans la direction longitudinale (8) sont dimensionnées de telle sorte que le rapport des étendues (Q/B) est de 1,0 < Q/B < 2,0.

7. Couche jetable pour l'incontinence selon une ou plusieurs des revendications précédentes, **caractérisée en ce que** les deux sections latérales arrière (22) présentent dans la direction longitudinale (8) une distance (d) par rapport à un bord transversal arrière (35) de la partie principale (4) d'au moins 1 mm, particulièrement d'au moins 5 mm, particulièrement d'au moins 10 mm, particulièrement d'au moins 15 mm, particulièrement d'au plus 50 mm.

8. Couche jetable pour l'incontinence selon une ou plusieurs des revendications précédentes, **caractérisée en ce qu'**une ligne droite (45) étendue dans la direction transversale et tangente au moyen de fermeture respectif sur le côté tourné vers l'entrejambe coupe le corps absorbant (6).

9. Couche jetable pour l'incontinence selon une ou plusieurs des revendications précédentes, **caractérisée en ce que** chaque section latérale arrière (22) comprend exactement un moyen de fermeture (28).

10. Couche jetable pour l'incontinence selon une ou plusieurs des revendications précédentes, **caractérisée en ce que** les sections latérales arrière (22) sont repliées sur elles-mêmes par le fabricant autour d'au moins deux axes de pliage de section latérale (46, 48) s'étendant dans la direction longitudinale (8) et des zones partielles (50, 52, 54) des sections latérales arrière (22) repliées l'une sur l'autre sont définies et délimitées par ces axes de pliage de section latérale (46, 48), et **en ce qu'**une zone partielle (54) située à l'extérieur dans la direction transversale (10) est configurée sous forme essentiellement inextensible.

11. Couche jetable pour l'incontinence selon la revendication 10, **caractérisée en ce qu'**une zone partielle (52) se raccordant vers l'intérieur à la zone partielle située à l'extérieur (54) est configurée sous forme inextensible sur au moins 40 % de sa surface à partir de l'axe de pliage extérieur (48) s'étendant dans la direction longitudinale.

12. Couche jetable pour l'incontinence selon la revendication 10 ou 11, **caractérisée en ce qu'**un axe de pliage de section latérale respectif (46) situé à l'intérieur à l'état déplié, c'est-à-dire adjacent au bord longitudinal latéral arrière (18) de la partie principale (4), s'étend à l'intérieur de la zone élastique ou rendue élastique (42) de la section latérale concernée (22).

13. Couche jetable pour l'incontinence selon la revendication 10, 11 ou 12, **caractérisée en ce qu'**un axe de pliage de section latérale respectif (48) situé plus à l'extérieur dans la direction transversale (10) à l'état déplié s'étend à l'intérieur d'une zone inextensible des sections latérales arrière (22).

14. Couche jetable pour l'incontinence selon une ou plusieurs des revendications précédentes, **caractérisée en ce que** les sections latérales arrière (22) sont repliées sur elles-mêmes par le fabricant autour d'exactement deux axes de pliage de section latérale (46, 48) s'étendant dans la direction longitudinale (8), de telle sorte qu'exactement trois zones partielles (50, 52, 54) des sections latérales (22) sont formées, et **en ce que** la zone partielle centrale (52) est configurée sous forme inextensible sur au moins 40 % de sa surface à partir de l'axe de pliage extérieur (48) s'étendant dans la direction longitudinale (8).

15. Couche jetable pour l'incontinence selon une ou plusieurs des revendications 10 à 14, **caractérisée en ce qu'**une étendue (A) des sections latérales arrière (22) repliées sur elles-mêmes dans la direction transversale (10) au-delà du bord longitudinal latéral arrière respectif (18) et une étendue B des sections latérales arrière (22) repliées sur elles-mêmes dans la direction longitudinale (8) sont dimensionnées de telle sorte que le rapport des étendues (A/B) est de 0,5 < A/B < 1.

16. Couche jetable pour l'incontinence selon une ou plusieurs des revendications 10 à 15, **caractérisée en ce que** la partie principale (4) est repliée sur elle-même vers l'intérieur par le fabricant, avec les sections latérales arrière (22) repliées sur elles-mêmes, autour d'un premier et d'un deuxième axe de pliage de partie principale s'étendant chacun dans la direction longitudinale (8), de telle sorte que les sections latérales arrière (22) des deux côtés viennent se situer en chevauchement au moins partiel l'une par rapport à l'autre dans la direction de l'épaisseur.

17. Couche jetable pour l'incontinence selon une ou plusieurs des revendications précédentes, **caractérisée en ce que** le corps absorbant contient un matériau polymère superabsorbant (SAP), particulièrement à hauteur de 5 à 100 pour cent en poids, préférentiellement à hauteur de 10 à 95 pour cent en poids, plus préférentiellement à hauteur de 15 à 90 pour cent en poids et de manière tout particulièrement préférée à hauteur de 20 à 80 pour cent en poids.
